# EUROPÄISCHE PATENTSCHRIFT

(11) **EP 0 531 483 B1**
(45) Veröffentlichungstag und Bekanntmachung des Hinweises auf die Patenterteilung: **23.08.1995**
(21) Anmeldenummer: 92906880.7
(22) Anmeldetag: 19.03.1992
(51) Int. Cl.: A61K 6/093, A61K 6/083, C08G 77/58, C08G 77/22

(54) **VERWENDUNG VON ZUSAMMENSETZUNGEN AUF DER BASIS VON ORGANISCH MODIFIZIERTEN KIESELSÄURE-POLYKONDENSATEN FÜR DIE BESCHICHTUNG VON ZÄHNEN UND ZAHNERSATZTEILEN**
USE OF COMPOSITIONS BASED ON ORGANICALLY MODIFIED POLYCONDENSATES OF SILICIC ACID FOR COATING TEETH AND TOOTH REPLACEMENT PARTS
UTILISATION DE COMPOSITIONS A BASE DE POLYCONDENSES D'ACIDE SILICIQUE ORGANIQUEMENT MODIFIES POUR LE REVETEMENT DE DENTS ET DE PROTHESES DENTAIRES

(30) Priorität: 19.03.1991 DE 9103321 U
(43) Veröffentlichungstag der Anmeldung: 17.03.1993
(73) Patentinhaber: THERA Patent GmbH & Co. KG Gesellschaft für industrielle Schutzrechte, D-82229 Seefeld (DE)
(72) Erfinder: SCHMIDT, Monika, D-6908 Jena (DE)
(74) Vertreter: Abitz, Walter, Dr.-Ing.
(86) Internationale Anmeldenummer: EP9200604
(87) Internationale Veröffentlichungsnummer: WO9216183

(56) Entgegenhaltungen:
- EP-A- 0 078 548
- EP-A- 0 358 011
- EP-A- 0 394 797
- PATENT ABSTRACTS OF JAPAN vol. 004, no. 159 (C-030)6. November 1980

## Beschreibung

Die Erfindung betrifft die Verwendung von Zusammensetzungen auf der Basis von organisch modifizierten Kieselsäure-Polykondensaten für die Beschichtung von Zähnen und Zahnersatzteilen.

Zähne und aus den verschiedensten Materialien gefertige Zahnersatzteile unterliegen der Gefahr der Anlagerung von Plaque.

Aufgabe der Erfindung ist es, Zähne und Zahnersatzteile gegenüber der Anlagerung von Plaque zu schützen.

Erfindungsgemäß wird diese Aufgabe gelöst durch die Verwendung von Zusammensetzungen auf der Basis von organisch modifizierten Kieselsäure-Polykondensaten. Diese Zusammensetzungen werden auf die Zähne oder Zahnersatzteile aufgebracht und gehärtet. Die Haftung ist außergewöhnlich gut, ein Haftvermittler ist nicht notwendig.

Überraschenderweise hat sich herausgestellt, daß Zusammensetzungen auf der Basis von organisch modifizierten Kieselsäure-Polykondensaten nach ihrer Härtung Überzüge auf den Zähnen und den Zahnersatzteilen geben, die gegenüber der Anlagerung von Plaque resistent sind. Dies ist deshalb überraschend, da in der deutschen Patentschrift 27 58 414 Kieselsäure-Polykondensat-Zusammensetzungen beschrieben sind als Beschichtungen von Trägermaterialien, z.B. Glasgefäßen für die Züchtung von Gewebe- und Zellkulturen.

In Spalte 3, Zeilen 32 ff. dieser Patentschrift heißt es, daß die Zellen hierbei - vermutlich durch chemische Bindungskräfte - fest auf der Unterlage gebunden sind, so daß ein Wachstum im Zellverbund gewährleistet ist. Man hätte demnach erwarten müssen, daß Überzüge auf der Basis von Kieselsäure-Polykondensat für die vorliegenden Zwecke vollkommen ungeeignet sind.

Die erfindungsgemäß einzusetzenden Zusammensetzungen auf der Basis von organisch modifizierten Kieselsäure-Polykondensaten umfassen
(a) mindestens eine im Reaktionsmedium lösliche Verbindung der Formel (I)

   MRₓ (I),

   in der M Ti, Zr, Si, Ge, Sn oder Al bedeutet, R Halogen, Hydroxy, Alkoxy, Acyloxy oder einen Chelatliganden darstellt und x die Wertigkeit von M bedeutet, sowie
(b) ein organisches Silan der Formel (II)

   R''ₙSiX₄₋ₙ (II)

   in der R'' Alkyl, Alkenyl, Aryl, Arylalkyl, Alkylaryl, Arylalkenyl oder Alkenylaryl bedeutet, wobei diese Reste durch Sauerstoff- oder Schwefelatome oder -NH-Gruppen unterbrochen sein können, X Wasserstoff, Halogen, Hydroxy, Alkoxy, Acyloxy oder die Gruppe -NR₂' (R' = Wasserstoff und/oder Alkyl) darstellt und n den Wert 1, 2 oder 3 hat,
und/oder
(c) ein organofunktionelles Silan der Formel (III)

   Rₘ''(R'''Y)ₙSiX₍₄₋ₘ₋ₙ₎ (III),

   in der R'', X und n die oben gegebene Bedeutung haben, R''' Alkylen, Phenylen, Alkylenphenylen oder Alkenylen bedeutet, wobei diese Reste durch Sauerstoff- oder Schwefelatome oder -NH-Gruppen unterbrochen sein können, Y Halogenatome, Hydroxy-, Mercapto-, Polyol-, z.B. Glycyl- oder Glyceryl-, gegebenenfalls substituierte Amino-, quarternäre Ammonium-, Amid-, Polyamid-, Aldehyd-, Keto-, Carboxy-, Carbonsäurealkylester-, Sulfonsäure-, Phosphorsäure-, Epoxy-, Acryloxy- oder Methacryloxygruppen darstellen und m den Wert 0, 1 oder 2 hat.

Die Zusammensetzungen können gegebenenfalls noch eine weitere Komponente (d) enthalten:
(d) im Reaktionsmedium lösliche, schwer-flüchtige Oxide eines Elements der Hauptgruppen Ia bis Va oder der Nebengruppen IVb oder Vb des Periodensystems, mit Ausnahme von Titan, Zirkon, Silicium, Germanium, Zinn und Aluminium, oder im Reaktionsmedium lösliche, unter den Reaktionsbedingungen ein schwer-flüchtiges Oxid bildende Verbindungen eines dieser Elemente.

Zusammensetzungen, die die Komponenten (a), (b) und (c) und gegebenenfalls (d) enthalten, sind aus der europäischen Patentschrift 78 548 bekannt. Deren gesamter Offenbarungsgehalt soll hier mitumfaßt sein.

In den vorstehenden Formeln (I), (II) und (III) können mehrmals vorhandene Reste R, R', R'', R''', X bzw. Y bei einer Verbindung jeweils die gleiche oder unterschiedliche Bedeutung haben.

Die Alkylreste bedeuten z.B. geradkettige, verzweigte oder cyclische Reste mit 1 bis 20, vorzugsweise 1 bis 10 Kohlenstoffatomen und insbesondere niedere Alkylreste mit 1 bis 6, vorzugsweise 1 bis 4 Kohlenstoffatomen. Spezielle Beispiele sind Methyl, Ethyl, n-Propyl, Isopropyl, n-Butyl, sek.-Butyl, tert.-Butyl, Pentyl, n-Hexyl und Cyclohexyl. Die Arylreste enthalten z.B. 6 bis 25, vorzugsweise 6 bis 14 und insbesondere 6 bis 10 Kohlenstoffatome. Spezielle Beispiele sind Phenyl und Naphthyl, wobei Phenyl bevorzugt ist.

Die Alkenylreste sind z.B. geradkettige, verzweigte oder cyclische Reste mit 2 bis 20, vorzugsweise 2 bis 10 Kohlenstoffatomen und insbesondere niedere Alkenylreste, wie Vinyl, Allyl und 2-Butenyl.

Die Alkoxy-, Acyloxy-, Alkylamino-, Arylalkyl-, Alkylaryl-, Arylalkenyl-, Alkenylaryl-, Alkylen-, Alkylenphenylen-, Keto-, Carbonsäurealkylester- und substituierten Aminoreste leiten sich z.B. von den vorstehend genannten Alkyl-, Alkenyl- und Arylresten ab. Spezielle Beispiele sind Methoxy, Ethoxy, n- und i-Propoxy, n-, sek.- und tert.-Butoxy, Acetyloxy, Propionyloxy, Monomethylamino, Monoethylamino, Dimethylamino, Diethylamino, Monomethylanilino, Benzyl, Tolyl, Methylen, Ethylen, Dimethylen, Toluylen und Styryl.

Die genannten Reste können gegebenenfalls übliche Substituenten tragen, z.B. Halogenatome, niedere Alkylreste, Hydroxy-, Nitro- oder Aminogruppen.

Unter den Halogenen sind Fluor, Chlor und Brom bevorzugt und Chlor besonders bevorzugt.

Spezielle Beispiele für Titan- oder Zirkonverbindungen der Komponente (a) sind TiCl₄, ZrCl₄, Ti(OC₂H₅)₄), Ti(Oi-C₃H₇)₄, Ti(OC₄H₉)₄, Ti(kresyl)₄, Zr(OC₃H₇)₄, Zr(OC₄H₉)₄, Ti(acetylacetonato)₂(Oi-C₃H₇)₂, Zr(acetylacetonato)₄ und andere Titan- oder Zirkonkomplexe mit Chelatliganden, die vorzugsweise über Sauerstoff und/oder Stickstoff koordiniert sind. Spezielle Beispiele für Silicium-, Germanium-, Zinn- und Aluminiumverbindungen der Komponente (a) sind Si(OH)₄, Si(OC₂H₅)₄, Si(OCH₃)₄, Si(OC₄H₉)₄, Si(OC₃H₇)₄, SiCl₄, GeCl₄, Ge(OC₂H₅)₄, Sn(OC₂H₅)₄, AlCl₃, Al(OC₂H₅)₃, Al(OC₃H₇)₄, Al(OC₄H₉)₃, Al(OH)₃.

Bevorzugte organische Silane (b) sind z.B.:
(CH₃)₂-Si-Cl₂, (CH₃)₂-Si-(OCH₃)₂, (CH₃)₂-Si-(OC₂H₅)₂, (C₆H₅)₂-Si-Cl₂, (C₆H₅)₂-Si-(OC₂H₅)₂, CH₂=CH-Si-Cl₃, CH₂=CH-CH₂-Si-(OC₂H₅)₃, CH₂=CH-CH₂-Si-(CH₃COO)₃, (i-C₃H₇)₃-Si-OH, (CH₃)₂-Si-(OH)₂ und (C₆H₅)₂-Si-(OH)₂. Diese Silane sind zum Teil Handelsprodukte oder sie lassen sich nach bekannten Methoden herstellen; vgl. W. Noll, "Chemie und Technologie der Silicone", Verlag Chemie GmbH, Weinheim/Bergstrasse (1968).

Bei den organofunktionellen Silanen (c) kann die Brückengruppe R''' gegebenenfalls durch Sauerstoff- oder Schwefelatome oder -NH-Gruppen unterbrochen sein. Vorzugsweise entstehen auf diese Art 2 bis 10 sich wiederholende Struktureinheiten.

Bevorzugte organofunktionelle Silane sind z.B.:
(C₂H₅O)₃-Si-(CH₂)₃-OH, (C₂H₅O)₃-Si-CH₂-NH₂, (CH₃O)₃-Si-(CH₂)₂-NH-(CH₂)₂-NH₂, (C₂H₅O)₃-Si-p-C₆H₄-NH₂, (C₂H₅O)₃-Si-(CH₂)₃-OH, (CH₃O)₃-Si-(CH₂)₄-SH, CH₃(CH₃O)₂-Si-CH₂-CH(CH₃)-CH₂-NH-(CH₂)₂-NH₂, CH₃(C₂H₅O)₂-Si-(CH₂)₄-NH₂, (CH₃)₂C₂H₅O-Si-CH₂-NH₂, CH₃(C₂H₅O)₂-Si-CH₂-OH, (CH₃-CH₂-O)₃-Si-CH₂-CH(CH₃)-CH₂-NH-(CH₂)₂-NH₂, (CH₃-CH₂-CH₂-O)₃-Si-(CH₂)₄-NH₂, (C(CH₃)₂(C₂H₅)O)₃-Si-CH₂-NH₂,

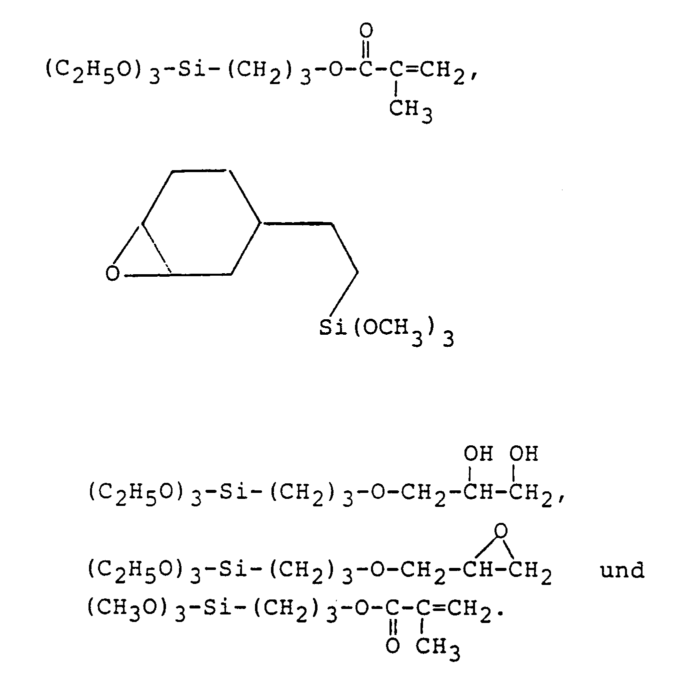

Bei den organischen Silanen (b) und den organofunktionellen Silanen (c) haben m vorzugsweise den Wert O und n vorzugsweise den Wert 1 oder 2.

Anstelle der monomeren Ausgangssilane (b) und (c) können gegebenenfalls auch vorkondensierte, im Reaktionsmedium lösliche Oligomere dieser Silane eingesetzt werden; d.h. geradkettige oder cyclische, niedermolekulare Teilkondensate (Polyorganosiloxane) mit einem Kondensationsgrad von z.B. etwa 2 bis 6.

Als Komponente (d) werden im Reaktionsmedium lösliche, schwer-flüchtige Oxide oder derartige schwer-flüchtige Oxide bildende Verbindungen von Elementen der Hauptgruppen Ia bis Va oder der Nebengruppen IVb oder Vb des Periodensystems eingesetzt. Vorzugsweise leitet sich die Komponente (d) von folgenden Elementen ab: Li, Na, K, Mg, Ca, B, Pb, P, As und/oder V, wobei Na, Ca, Mg, Sr, B und P besonders bevorzugt sind.

Unter den schwer-flüchtigen Oxiden sind Li₂O, Na₂O, K₂O, CaO, MgO, As₂O₃, P₂O₅ und B₂O₃ besonders bevorzugt.

Im Reaktionsmedium lösliche, schwer-flüchtige Oxide bildende Verbindungen sind z.B. anorganische Säuren, wie Phosphorsäure und Borsäure, sowie deren Ester. Ferner eignen sich z.B. Halogenide, wie SiCl₄ und HSiCl₃, und Alkoxide, wie NaOR, KOR, Ca(OR)₂, wobei sich R von niederen Alkoholen, wie Methanol, Ethanol, Propanol oder Butanol, ableitet. Weitere verwendbare Ausgangsverbindungen sind entsprechende Salze mit flüchtigen Säuren, z.B. Acetate, basische Acetate, wie basisches Bleiacetat, und Formiate.

Die Zusammensetzungen umfassen 1 bis 99, vorzugsweise 20 bis 90, insbesondere 40 bis 80 Mol-% an Komponente (a) und entsprechend 1 bis 99, vorzugsweise 10 bis 80 und insbesondere 20 bis 60 Mol-% an Komponente (b), oder
die Zusammensetzungen umfassen 1 bis 99, vorzugsweise 20 bis 90, insbesondere 40 bis 60 Mol-% an Komponente (a) und entsprechend 1 bis 99, vorzugsweise 10 bis 80 und insbesondere 20 bis 60 Mol-% an Komponente (c), oder
die Zusammensetzungen umfassen 1 bis 98, vorzugsweise 20 bis 80, insbesondere 40 bis 60 Mol-% an Komponente (a), 1 bis 98, vorzugsweise 10 bis 75 und insbesondere 15 bis 50 Mol-% an Komponente (b) und 1 bis 40, vorzugsweise 5 bis 30 und insbesondere 10 bis 25 Mol-% an Komponente (c).

Bei Einsatz der Komponente (d) enthalten die Zusammensetzungen 0,1 bis 50, vorzugsweise 0,5 bis 30 und insbesondere 2 bis 20 Gew.-% an Komponente (d), bezogen auf das Gesamtgewicht der Ausgangskomponenten (a) bis (d).

Die vorzugsweise wasserfreien Ausgangskomponenten werden zunächst im gewünschten Mengenverhältnis, gegebenenfalls in Gegenwart eines wasserfreien organischen Lösungsmittels und gegebenenfalls in Anwesenheit eines wasserfreien Kondensationskatalysators, vorkondensiert. Beispiele für geeignete Lösungsmittel sind Alkohole, vorzugsweise niedere Alkohole, wie Methanol, Ethanol oder Butanol, Ether, vorzugsweise niedere Dialkylether, wie Diethylether oder Dioxan, Ketone, z.B. Aceton, Ester, Benzol und deren Gemische. Die Menge an Lösungsmittel beträgt 0 bis 90, vorzugsweise 40 bis 70 Gew.-%, bezogen auf das Gewicht der Komponenten (a) bis (d).

Als Kondensationskatalysatoren für die wassersfreie Vorkondensation verwendet man wasserfreie Säuren und Basen. Als Säuren kommen insbesondere flüchtige Säuren, wie Halogenwasserstoffe oder Eisessig, die gegebenenfalls in einem der vorstehenden wasserfreien organischen Lösungsmittel gelöst sind, anorganische Säuren, wie Schwefelsäure, Aluminiumtrichlorid oder Bortrifluorid, oder organische Säuren, wie Ameisensäure, Essigsäure oder Propionsäure, in Frage. Bei Verwendung von alkoholischen Lösungen flüchtiger Säuren, wie methanolischer 1 N HCl, kann die Gesamt-Katalysatorkonzentration z.B. bis zu 50 Molprozent betragen, wobei die Zugabe vorzugsweise in Einzelportionen erfolgt.

Als Basen kommen anorganische und organische Basen, wie Ammoniak, Natrium-, Kalium- oder Calciumhydroxid oder Trialkylamine, in Frage, wobei flüchtige Basen, wie Ammoniak oder Trialkylamine, besonders bevorzugt sind.

Die Vorkondensation wird üblicherweise bei Temperaturen von -20 bis 200°C, vorzugsweise 50 bis 150°C und insbesondere bei der Siedetemperatur des Lösungsmittels durchgeführt.

Die Vorkondensation kann auch in Anwesenheit von Wasser auf verschiedenen Wegen erfolgen:
1) Partielle Hydrolyse:
   Zugabe von unterstöchiometrischen Wassergehalten zur reaktionsträgsten Komponente. Anschließend werden die hydrolysereaktiveren Komponenten hinzugefügt. Die Wasserzugabe kann in unterschiedlich schonender Form zur Komponente (a) erfolgen, z.B.
   - gelöst in Alkohol,
   - Zugabe des Wassers durch eine feuchte Atmosphäre,
   - Erzeugung des Wassers in der Reaktionslösung durch Esterbildung,
   - Zugabe von Salzhydraten.
2) Zugabe von die Hydrolyse kontrollierenden Verbindungen, von z.B. 2,5-Pentandion, Essigsäure, Acetessigsäureethylester und dergleichen,
3) Zugabe von die Trocknung steuernden Mittel, wie z.B. Oxalsäure, Formamid und dergleichen.

Gegebenenfalls kann man zunächst einen Teil einer, mehrerer oder aller Ausgangskomponenten vorkondensieren, dann die übrigen Ausgangskomponenten zumischen und anschließend hydrolytisch oder nicht-hydrolytisch cokondensieren.

Im allgemeinen wird die Vorkondensation soweit durchgeführt, daß das entstehende Vorkondensat noch flüssige Konsistenz hat.

Das Oligomere oder niedermolekulare, partiell hydrolysierte und kondensierte anorganisch-organische Vorkondensat kann dann isoliert und in einem organischen Lösungsmittel zu einer Zusammensetzung definierter Viskosität gelöst werden. Als Lösungsmittel sind höhere Alkohole, Ester, z.B. Essigsäureethyl- oder -amylester, Toluol, Chloroform, Aceton u.a. geeignet. Die Viskosität der Beschichtungszusammensetzung kann zu einem Wert im Bereich von 5 bis 80 mPa·s eingestellt werden, der von dem ausgewählten Beschichtungsverfahren abhängig ist. Die Menge an Lösungsmittel beträgt im allgemeinen 20 bis 95, vorzugsweise 40 bis 80 Gew.-%, bezogen auf das Gewicht des Vorkondensats.

Darüber hinaus können noch Hilfsstoffe zugesetzt werden, wie z.B.

| | |
|---|---|
| Füllstoffe | 0 - 50 Gew.-% |
| Viskositätsregler | 0 - 15 Gew.-% |
| Konservierungsmittel | 0 - 2 Gew.-% |
| Pigmente | 0 - 2 Gew.-% |
| Verlauf- und Benetzungshilfsmittel | 0 - 5 Gew.-% |
| Stabilisatoren | 0 - 5 Gew.-% |
| Inhibitoren | 0 - 5 Gew.-% |

(jeweils bezogen auf das Gesamtgewicht der Komponenten (a) bis (d)), die eine individuelle Konsistenz- und Farbeinstellung der Mischungen ermöglichen.

Die auf diese Weise erhaltenen Lösungen werden dann auf die zu beschichtenden Dentalmaterialien oder Zähne durch Aufsprühen, Tauchen oder Aufpinseln aufgetragen. Bevorzugt sind dabei Tauchen oder Aufpinseln. Dabei kann nach Abdunsten des Lösungsmittels ein mehrfacher Auftrag angezeigt sein.

Anschließend wird bei Temperaturen zwischen etwa 36° und 120°C für einige wenige Minuten bis einige Stunden thermisch nachbehandelt und die Schicht verfestigt. Falls organofunktionelle Silane mit ethylenisch ungesättigten oder epoxyfunktionellen Gruppen zugegen sind, können diese auch über kationische oder radikalische Polymerisation vorverfestigt werden, bevor die weitere Polykondensation dann auch bei Raumtemperatur und über längere Zeit hinweg eintritt. Insbesondere ist es möglich, für den Fall von funktionellen Silanen mit ethylenisch ungesättigten Gruppen durch radikalische Polymerisation, insbesondere durch Photopolymerisation, eine erste Verfestigung der Schichten zu erreichen. Zu diesem Zweck werden den Zusammensetzungen kationische oder radikalische Initiatoren zugesetzt. Als kationische Initiatoren eignen sich beispielsweise Aryldiazoniumsalze, Diaryljodoniumsalze, Triarylsulfoniumalze sowie metallocenartige Komplexsalze. Eine für die erfindungsgemäßen Zwecke bevorzugte Initiatorenklasse sind die aus der EP-A 01 82 744 bekannten Metallocen-Komplexsalze, insbesondere die Verbindung (I):

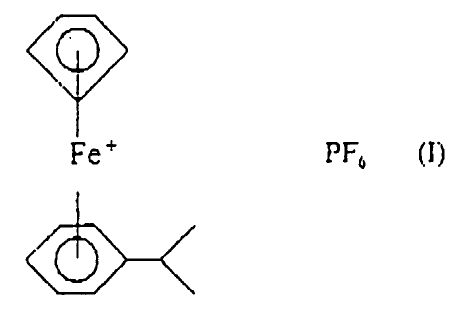

Eine weitere bevorzugte Klasse von Initiatoren für die kationische Polymerisation sind die Diaryljodoniumsalze der Formel:

Ar₂J⁺X⁻,

worin Ar ein gegebenenfalls substituiertes Aren ist, beispielsweise Benzol, Toluol, Xylol, Ethylbenzol, Methoxybenzol, Naphthalin, 1,2-Dihydronaphthalin, Phenanthren, Anthracen, 9,10-Dihydroanthracen, Diphenylen, Biphenyl, Cumol; und worin X⁻ ein komplexes Anion, beispielsweise BF₄⁻, PF₆⁻, AsF₆⁻, SbF₆⁻, vorzugsweise BF₄⁻ oder PF₆⁻, ist. Besonders bevorzugt sind Diphenyljodonium-tetrafluoroborat, Ditoluyljodonium-tetrafluoroborat, Ditoluyljodonium-hexafluorophosphat und Di-t-butylphenyljodonium-tetrafluoroborat. Weitere geeignete Diaryljodoniumsalze finden sich beispielsweise in "UV-Curing", Science and Technology, Ed. by S. Peter Pappas, Technology Marketing Corporation, Norwalk, USA, 06851 (1980).

Beispiele für geeignete Radikalbildner sind organische Peroxide, z.B. Diacylperoxide, wie Benzoylperoxid und Lauroylperoxid; Ketoperoxide, wie Acetonperoxid und Cyclohexanonperoxid, Kohlenwasserstoffperoxide, wie tert.-Butylhydroperoxid, Cumolhydroperoxid und Dekahydronaphthalinhydroperoxid; Dikohlenwasserstoffperoxide, wie Di-tert.-butylperoxid und Dicumylperoxid; Perketale, wie 1,1-Di-tert.-butylperoxy-3,3,5-trimethylcyclohexan; Perester, wie tert.-Butylperbenzoat, tert.-Butylperoxyisopropylpercarbonat, tert.-Butylperpivalat, tert.-Butylperoctoat, tert.-Butylcyclohexylpercarbonat und tert.-Butylpermaleinat, sowie Acetylcyclohexansulfonylperoxid. Ebenfalls geeignet sind übliche Azoinitiatoren, wie Azobisisobutyronitril.

Als Photoinitiatoren kommen sämtliche, für die Härtung von lichthärtenden Kunststoffen bekannten Photoinitiatoren in Betracht. Geeignete Photoinitiatoren, die nach Bestrahlen durch UV oder sichtbares Licht die Polymerisation auslösen, sind beispielsweise Benzoinalkylether, Benzilmonoketale, Acylphosphinoxide oder aliphatische und aromatische 1,2-Diketo-Verbindungen (zum Beispiel Campherchinon).

Zur Beschleunigung der Polymerisation können zusammen mit den Photoinitiatoren sogenannte Aktivatoren, wie Amine oder organische Phosphite, zugesetzt werden.

Die Belichtung des aufgetragenen Materials erfolgt auf übliche apparative Weise. Es ist nur darauf zu achten, daß die Lichtquelle auf den verwendeten Photoinitiator abgestimmt ist, das heißt, daß die Wellenlänge des emitierten Lichtes dem Absorptionsverhalten des Photoinitiators entspricht. Mischungen von Photoinitiatoren, die einen breiteren Absorptionsbereich abdecken, machen das Verfahren etwas unabhängiger von der jeweils einzusetzenden Lichtquelle.

Die Initiatoren werden vorzugsweise in Mengen von 0,05 bis 3 Gew.-%, bezogen auf das Gewicht der in den Zusammensetzungen vorhandenen ungesättigten Verbindungen, eingesetzt.

Die ausgehärteten Überzüge sind resistent gegenüber Plaque-Anlagerung. Sie zeichnen sich im übrigen durch homogene Oberflächen aus und beeinträchtigen die Farbe des Basismaterials wegen ihrer Transparenz nicht. Sie sind kratz- und abriebfest, hydrolysebeständig und weisen eine gute Oberflächenhärte auf.

Die erfindungsgemäßen Überzüge eignen sich zur Beschichtung von Zähnen sowie Zahnersatzteilen, die im Mundmilieu der Gefahr der Plaque-Anlagerung unterliegen. Besonders geeignet sind die Beschichtungen für Kunststoffmaterialien wie Prothesenbasisplatten, Teilprothesen, künstliche Zähne, Composite-Füllungen, Kunststoff-Inlays und insbesondere Kunststoffverblendungen.

Bei einer besonderen Ausführungsform der Erfindung können die erfindungsgemäßen Vorkondensate auch mit ethylenisch ungesättigten, copolymerisierbaren Monomeren abgemischt werden, die mit dem Vorkondensat homogen mischbar oder darin löslich sind. Geeignete Monomeren sind beispielsweise (Meth)acrylsäure und deren Salze, vorzugsweise die Alkalimetallsalze, wie das Natriumsalz; (Meth)acrylsäureester, wie Methyl(meth)acrylat, Ethyl(meth)acrylat, Butyl(meth)acrylat, Cyclohexyl(meth)acrylat, Glycidyl(meth)acrylat, Ethylenglykolmono(meth)acrylat, Diethylenglykolmono(meth)acrylat, Triethylenglykolmono(meth)acrylat, Allyl(meth)acrylat, 2-Hydroxypropyl(meth)acrylat, 3-Hydroxypropyl(meth)acrylat, 2-Ethoxyethyl(meth)acrylat, 2-Dimethylaminoethyl(meth)acrylat und 3-Methoxy-2-hydroxypropyl(meth)acrylat; (Meth)acrylsäureamide, wie (Meth)acrylamid, N-Methylol(meth)acrylamid und Dimethyl(meth)acrylamid; Allylverbindungen, wie Allylalkohol und Allylglycidylether; N-Vinylpyrrolidon, und Styrol. Unter diesen Monomeren sind Alkyl(meth)acrylsäureester, Alkoxyalkyl(meth)acrylsäureester und Hydroxyalkyl(meth)acrylsäureester mit 1 bis 6 Kohlenstoffatomen in der Alkyl- bzw. Alkoxygruppe sowie N-Vinylpyrrolidon besonders bevorzugt. Die Menge an Monomeren kann 1 bis 50 Gew.-%, vorzugsweise 10 bis 30 Gew.-%, bezogen auf das Gesamtgewicht aus Vorkondensat und Monomeren, betragen.

Gegebenenfalls kann man die Polymerisation bzw. Copolymerisation in Gegenwart einer oder mehrerer ungesättigter Verbindungen als Vernetzungsmittel durchführen. Spezielle Beispiele für geeignete Vernetzungsmittel sind Ethylenglycoldi(meth)acrylat, Diethylenglykoldi(meth)acrylat, Triethylenglykoldi(meth)acrylat, Butylenglykoldi(meth)acrylat, Neopentylglykoldi(meth)acrylat, Pentaerythrittri(meth)acrylat, Pentaerythrittetra(meth)acrylat, Allyl(meth)acrylat, Trimethylolpropantri(meth)acrylat, Divinylbenzol und Diallylphthalat.

Geeignet sind ebenfalls die langkettigen Monomeren gemäß US-Patent 3 066 112 auf der Basis von Bisphenol-A und Glycidylmethacrylat oder deren durch Addition von Isocyanaten entstandenen Derivate. Geeignet sind auch Verbindungen des Typs Bisphenol-A-diethyl(meth)acrylat und Bisphenol-A-dipropyl(meth)acrylat. Weiterhin geeignet sind die mit Alkoxydeinheiten verlängerten Derivate der genannten Bisphenol-A-Typen, wie beispielsweise die Diacryl- bzw. Dimethacrylsäureester von Bishydroxypolyalkoxybisphenol-A-Derivaten.

Geeignet sind weiterhin auch die in dem deutschen Patent 2 816 823 genannten Diacryl- und Dimethacrylsäureester des Bishydroxymethyltricyclo[5.2.1.0^{2,6}]-decans und der mit Alkoxyd verlängerten Derivate des Bishydroxymethyltricyclo[5.2.1.0^{2,6}]-decans. Verwendet werden können auch die Reaktionsprodukte aus Diisocyanaten und Hydroxyalkyl(meth)acrylaten, wie sie in der deutschen Offenlegungsschrift 23 12 559 beschrieben sind.

Die Menge an Vernetzungsmittel beträgt vorzugsweise 1 bis 50 Molprozent, insbesondere 10 bis 30 Molprozent, bezogen auf die Gesamtmolzahl der Monomeren.

Den Monomeren werden je nach Wahl der Härtungsbedingungen Initiatoren der oben angegebenen Art in den ebenfalls angegebenen Mengenverhältnissen hinzugegeben. Die Aufbringung der Überzüge und deren Härtung erfolgt dann analog zu der oben geschilderten Weise.

Bei einer besonderen Ausführungsform der Erfindung eignen sich als kationisch härtbare Harze oder kationisch polymerisierbare Monomere beispielsweise mono- oder polyfunktionelle Vinylether und Vinylester. Geeignete Vinylether sind Trimethylolpropan-Trivinylether, Ethylenglykol-Divinylether und cyclische Vinylether. Besonders geeignet ist Triethylenglykoldivinylether.

Allgemein gut geeignete Verbindungen sind die Vinylester und Vinylether polyfunktioneller Alkohole, wobei Polyethylen- und Polypropylenglykole mit Vinylether-Endgruppen bevorzugt eingesetzt werden.

Weiterhin gut geeignet sind kationisch polymerisierbare heterocyclische Verbindungen, beispielsweise Epoxide. Bevorzugt verwendet werden hierbei die Glycidylether ein- oder mehrwertiger Alkohole, beispielsweise die Diglycidylether von Bisphenol-A. Zur Einstellung einer hohen Reaktivität sind besonders geeignet die Di- und Polyepoxide cycloaliphatischer Verbindungen, beispielsweise die Glycidylether und β-Methylglycidylether cycloaliphatischer Diole und Polyole.

Verwendbar sind ferner als Glycidylverbindungen die Glycidylester von Carbonsäuren, insbesondere von Di- und Polycarbonsäuren, beispielsweise die Glycidylester von Bernsteinsäure, Adipinsäure, Phthalsäure.

Beispiele für besonders reaktive Glycidylverbindungen sind die Diepoxide des Vinylcyclohexans und des Dicyclopentadiens sowie 3-(3',4'-Epoxycyclohexyl)-8,9-epoxy-2,4-dioxyspiro-(5,5)undecan und 3,4-Epoxycyclohexylmethyl-3',4'-erpoxycyclohexylcarboxylat.

Bevorzugte Epoxidharze sind gegebenenfalls vorverlängerte und/oder prepolymere Diglycidylether zweiwertiger Phenole oder zweiwertiger aliphatischer Alkohole mit zwei bis vier C-Atomen. Besonders bevorzugt verwendet werden die vorverlängerten Diglycidylether des 2,2-bis-(4-Hydroxyphenyl)propans.

### HERSTELLUNGSBEISPIELE

### Beispiel 1

13,68 g Tetraethoxytitanat werden in 50 ml einer Mischung von Toluol und Ethanol (1:1) gelöst und 0,6 ml Eisessig hinzugefügt. Diese Mischung wird 0,5 h bei 50°C gerührt. Getrennt hiervon werden 30,28 g Diphenylsilandiol in einer Mischung von Toluol und Ethanol (1:1) gelöst. Diese Lösung wird langsam unter ständigem Rühren bei 50°C in die Tetraethoxytitanatlösung getropft und anschließend 1 h bei 75°C unter Rückflußbedingungen erhitzt. In die auf Raumtemperatur abgekühlte Lösung wird eine Mischung von 10 ml Wasser in 10 ml Ethanol unter Rühren hinzugetropft und 1 h bei Raumtemperatur gerührt. Unter reduziertem Druck werden Lösungsmittel und Wasser bei 60°C entfernt. Es entsteht ein gelbliches, transparentes, viskoses Vorkondensat.

### Beispiel 2

13,8 g Diphenyldichlorsilan werden in 17,1 ml Ethanol, 6,8 g Methylvinyldichlorsilan in 8,4 ml Ethanol und 0,53 g Tetraethoxysilan in 1,5 ml Ethanol gelöst. Die ethanolischen Lösungen werden vereinigt und 2 h unter Rückflußbedindungen bei 78°C erhitzt. Danach werden 7,2 ml 0,01 n Salzsäure langsam bei 70°C zugetropft und der Ansatz 0,5 h bei dieser Temperatur gehalten. Das Lösungsmittel und Wasser werden unter leichtem Vakuum entfernt. Das verbleibende Silan wird in Essigester zu einer 10 %igen Lösung gelöst und 0,2 g Dibenzoylperoxid zugefügt. Die Beschichtungen wurden bei Temperaturen zwischen 60 und 120°C getrocknet.

### Beispiel 3

13,1 g Diphenyldichlorsilan werden in 15,8 ml Ethanol, 6,5 g Methylvinyldichlorsilan in 7,9 ml Ethanol und 1,53 g Tetraethoxysilan in 3,0 ml Ethanol gelöst. Die Lösungen werden vereinigt und 2 h unter Rückflußbedingungen bei 78°C erhitzt. Dazu werden bei 70°C langsam 7,2 ml 0,01 n Salzsäure hinzugetropft und der Ansatz 0,5 h bei dieser Temperatur belassen. Unter leichtem Vakuum werden Lösungsmittel und Wasser abdestilliert. Das Silan wird zu einer 10 %igen Lösung in Essigester gelöst. Der Beschichtungslösung wurden 0,2 g Dibenzoylperoxid hinzugefügt. Die Beschichtungen wurden bei Temperaturen zwischen 60 und 120°C getrocknet.

### Beispiel 4

10,4 g Methacryloxypropyltrimethoxysilan und 3,8 g Methyltrimethoxysilan werden bei Raumtemperatur gemischt und unter Rühren 5,75 g Aluminium-sec.-butylat hinzugetropft. Die Mischung wird 10 min gerührt und auf 15°C abgekühlt. Dazu werden langsam 0,84 ml Wasser hinzugetropft, 10 min gerührt und die Lösung auf 10°C abgekühlt. Danach werden 1,68 ml Wasser langsam hinzugetropft und nach 15 min Rühren weitere 5 ml Wasser zugegeben. Das Produkt wird 2 h gerührt. Es entsteht eine klare Lösung, von der das Lösungsmittel unter leichtem Vakuum abdestilliert wird. Das verbleibende Produkt wird in 50 ml Essigester gelöst und der Ester mehrmals abdestilliert. Zum Schluß wird eine Lösung des Silans in 50 ml Essigester für Beschichtungen verwendet. Die Schichten wurden bei 90°C getrocknet.

### Beispiel 5

20 g Tetraethoxysilan werden in 50 ml Ethanol gelöst und 0,6 ml Eisessig hinzugegeben. Diese Mischung wird 30 Minuten bei 50°C gerührt und eine Lösung von 26,2 g Diphenylsilandiol in einer Mischung aus Toluol und Ethanol (1:1) hinzugegeben. Diese Lösung wird unter ständigem Rühren bei 50°C in die Tetraethoxysilanlösung getropft und wird anschließend 1 Stunde bei 75°C unter Rückflußbedingungen erhitzt. Zu dieser auf Raumtemperatur abgekühlten Lösung wird eine Mischung von 10 ml Wasser und 10 ml Ethanol unter Rühren hinzugefügt und 1 Stunde bei Raumtemperatur gerührt. Lösungsmittel und Wasser werden unter reduziertem Druck bei 60°C entfernt. Es entsteht ein farbloses, transparentes, viskoses Polymer, das für Beschichtungszwecke eingesetzt werden kann.

### ANWENDUNGSBEISPIELE - PLAQUE-ANLAGERUNGSUNTERSUCHUNGEN

### Beispiel 6

3,5 g des Vorkondensats von Beispiel 1 werden in 6,5 g Toluol gelöst. Mit einem Pinsel wird eine dünne Schicht auf den Kollocryl B - Prüfkörper aufgebracht und 24 h bei 95°C getrocknet. Man erhält eine glasklare, fest haftende Beschichtung.

### Beispiel 7

Die in Beispiel 2 erhaltene Lösung wird auf den Prüfkörper aufgestrichen und 12 h bei 80°C gehärtet.

### Beispiel 8

Die nach Beispiel 4 erhaltene Lösung wird mit 0,1 g Campherchinon sowie 0,2 g 2-Dimethylaminoethylmethacrylat versetzt. Nach dem Auftragen der Beschichtung auf die Prüfkörper wird 10 Minuten bei 40°C getrocknet. Mit einem handelsüblichen Kaltlichtgerät (Elipar II, Fa. ESPE) wird durch 40 Sekunden Belichtung die Schicht ausgehärtet.

### Meßprinzip:

Beschichtete Kunststoffprüfkörper wurden von Patienten 3 Tage getragen. Anschließend wurde durch Wägung der Prüfkörper die Plaque-Akkumulation in Abhängigkeit vom Beschichtungsverfahren bestimmt.

### Material:

PMMA (Prüfkörper aus Prothesenkunststoff Kallocryl B) Prüfkörpermaße: 5 x 7 x 1 mm

### Methode:

Die Prüfkörper wurden erfindungsgemäß beschichtet, im Exsikkator auf Gewichtskonstanz getrocknet und mit Hilfe einer Präzisionswaage ausgewogen. Anschließend wurden die Prüfkörper in einer Oberkieferminiplastschiene drei verschiedenen Probanden (1 weiblich, 2 männlich, Durchschnittsalter 21 Jahre) eingesetzt und 3 Tage im Mund getragen. Dabei wurden die Schienen nicht gereinigt, nur nach dem Essen grob mit Wasser abgespült. Nach der Tragezeit wurden die Prüfkörper dem Tragegerät entnommen, 24 Stunden im Exsikkator getrocknet und erneut ausgewogen. Tabelle 1 zeigt die Plaque-Anlagerung in Abhängigkeit des Beschichtungsverfahrens in »g/cm².
- Vergleichsbeispiel 1:: Prüfkörper unbeschichtet
- Vergleichsbeispiel 2:: Prüfkörper beschichtet mit herkömmlichem Prothesenlack auf Acrylatbasis (Palaseal, Firma Kulzer).

| Ergebnisse: Plaque-Anlagerung [»g/cm²] | | | | | |
|---|---|---|---|---|---|
| Proband | V 1 | V 2 | Beisp.6 | Beisp.7 | Beisp.8 |
| I | 1,94 | 1,74 | 0,47 | 0,68 | 0,42 |
| II | 1,87 | 1,68 | 0,31 | 0,55 | 0,38 |
| III | 1,98 | 1,81 | 0,53 | 0,48 | 0,78 |
| Durchschnittswerte | 1,93 | 1,74 | 0,44 | 0,57 | 0,54 |

Die erfindungsgemäßen Beispiele zeigen eine sehr stark verminderte Plaque-Anlagerung im Vergleich zum unbeschichteten Prüfkörper (V1).

Dagegen zeigt eine herkömmliche Oberflächenversiegelung auf Basis eines Acrylatlackes keine wesentliche Verminderung der Plaque-Anlagerung gegenüber den unbeschichteten Prüfkörpern. Darüber hinaus zeigte sich der interessante Effekt, daß die geringfügigen Plaquebeläge bei den erfindungsgemäß beschichteten Prüfkörpern sehr leicht entfernt werden konnte, während sie auf den Vergleichskörpern fest anhafteten.

In der Praxis kommt diesem Effekt besondere Bedeutung zu, da sich im Gegensatz zum Stand der Technik bei erfindungsgemäßer Beschichtung die Restbeläge durch die Zahnbürste leicht entfernen lassen.

## Patentansprüche

1. Verwendung von Zusammensetzungen, umfassend
(a) mindestens eine im Reaktionsmedium lösliche Verbindung der Formel (I)
MRₓ (I),
in der M Ti, Zr, Si, Ge, Sn oder Al bedeutet, R Halogen, Hydroxy, Alkoxy, Acyloxy oder einen Chelatliganden darstellt und x die Wertigkeit von M bedeutet, sowie
(b) ein organisches Silan der Formel (II)
R''ₙSiX₄₋ₙ (II)
in der R'' Alkyl, Alkenyl, Aryl, Arylalkyl, Alkylaryl, Arylalkenyl oder Alkenylaryl bedeutet, wobei diese Reste durch Sauerstoff- oder Schwefelatome oder -NH-Gruppen unterbrochen sein können, X Wasserstoff, Halogen, Hydroxy, Alkoxy, Acyloxy oder die Gruppe -NR₂' (R' = Wasserstoff und/oder Alkyl) darstellt und n den Wert 1, 2 oder 3 hat,
und/oder
(c) ein organofunktionelles Silan der Formel (III)
Rₘ''(R'''Y)ₙSiX₍₄₋ₘ₋ₙ₎ (III),
in der R'', X und n die oben gegebene Bedeutung haben, R''' Alkylen, Phenylen, Alkylenphenylen oder Alkenylen bedeutet, wobei diese Reste durch Sauerstoff- oder Schwefelatome oder -NH-Gruppen unterbrochen sein können, Y Halogenatome, Hydroxy-, Mercapto-, Polyol-, z.B. Glykyl- oder Glyceryl-, gegebenenfalls substituierte Amino-, quarternäre Ammonium-, Amid-, Polyamid-, Aldehyd-, Keto-, Carboxy-, Carbonsäurealkylester-, Sulfonsäure-, Phosphorsäure-, Epoxy-, Acryloxy- oder Methacryloxygruppen darstellen und m den Wert 0, 1 oder 2 hat,
zur Herstellung von Beschichtungen für Zahnersatzteile oder zur Herstellung von kosmetischen Beschichtungen von Zähnen.

2. Verwendung nach Anspruch 1, dadurch gekennzeichnet, daß die Zusammensetzungen 1 bis 99, vorzugsweise 20 bis 90, insbesondere 40 bis 80 Mol-% an Komponente (a) und entsprechend 1 bis 99, vorzugsweise 10 bis 80 und insbesondere 20 bis 60 Mol-% an Komponente (b) oder
1 bis 99, vorzugsweise 20 bis 90, insbesondere 40 bis 80 Mol-% an Komponente (a) und entsprechend 1 bis 99, vorzugsweise 10 bis 80 und insbesondere 20 bis 60 Mol-% an Komponente (c)
oder
1 bis 98, vorzugsweise 20 bis 80, insbesondere 40 bis 60 Mol-% an Komponente (a), 1 bis 98, vorzugsweise 10 bis 75 und insbesondere 15 bis 50 Mol-% an Komponente (b) und 1 bis 40, vorzugsweise 5 bis 30 und insbesondere 10 bis 25 Mol-% an Komponente (c) umfassen.

3. Verwendung nach den Ansprüchen 1 bis 2, dadurch gekennzeichnet, daß die Zusammensetzungen zusätzlich
(d) ein im Reaktionsmedium lösliches, schwer-flüchtiges Oxid eines Elements der Hauptgruppen Ia bis Va oder der Nebengruppen IVb oder Vb des Periodensystems, mit Ausnahme von Titan, Zirkon, Silicium, Germanium, Zinn und Aluminium, oder eine im Reaktionsmedium lösliche, unter den Reaktionsbedingungen ein schwerflüchtiges Oxid bildende Verbindung eines dieser Elemente
umfassen.

4. Verwendung nach Anspruch 3, dadurch gekennzeichnet, daß die Zusammensetzungen 0,1 bis 50, vorzugsweise 0,5 bis 30 und insbesondere 2 bis 20 Gew.-% an Komponente (d), bezogen auf das Gesamtgewicht der Ausgangskomponenten (a) bis (d), umfassen.

5. Verwendung nach den Ansprüchen 1 bis 4, dadurch gekennzeichnet, daß man die Ausgangskomponenten (a), (b) und/oder (c) und gegebenenfalls (d) in den gewünschten Mengenverhältnissen, gegebenenfalls unter Einsatz von geeigneten Katalysatoren vorkondensiert, das Vorkondensat isoliert und zur Herstellung von Beschichtungslösungen einer Viskosität von etwa 5 bis 80 MPa·s in einem organischen Lösungsmittel löst.

6. Verwendung nach Anspruch 5, dadurch gekennzeichnet, daß man die Beschichtungen mit einem ethylenisch ungesättigten copolymerisierbaren Monomeren, das mit der Vorkondensatlösung homogen mischbar oder darin löslich ist, und einem geeigneten Initiator abmischt.

7. Verwendung nach Anspruch 6, dadurch gekennzeichnet, daß die Menge an ethylenisch ungesättigten Monomeren/Vernetzer 1 bis 50, vorzugsweise 10 bis 30 Gew.-%, bezogen auf das Gesamtgewicht aus Vorkondensat und ethylenisch ungesättigten Monomeren/Vernetzer, und die Menge an Initiator 0,05 bis 3 Gew.-%, bezogen auf das Gewicht der ethylenisch ungesättigten Monomeren/Vernetzer, beträgt.

8. Verwendung der nach den Ansprüchen 5 bis 7 erhaltenen Beschichtungslösungen zur Herstellung von Beschichtungen auf Zahnersatzteilen, dadurch gekennzeichnet, daß man die Beschichtungslösungen auf die Zahnersatzteile aufträgt und anschließend bei Temperaturen von Zimmertemperatur bis 120°C, gegebenenfalls- unter Bestrahlung mit sichtbarem Licht oder UV-Licht, härtet.

9. Verwendung nach Anspruch 8, dadurch gekennzeichnet, daß man die Beschichtungslösungen auf Zahnersatzteile aus Kunststoff aufträgt.

10. Verwendung nach Anspruch 9, dadurch gekennzeichnet, daß man die Beschichtungslösungen auf Kunststoffverblendungen aufträgt.

11. Zusammensetzung für die therapeutische Beschichtung von Zähnen, umfassend
(a) mindestens eine im Reaktionsmedium lösliche Verbindung der Formel (I)
MRₓ (I),
in der M Ti, Zr, Si, Ge, Sn oder Al bedeutet, R Halogen, Hydroxy, Alkoxy, Acyloxy oder einen Chelatliganden darstellt und x die Wertigkeit von M bedeutet, sowie
(b) ein organisches Silan der Formel (II)
R''ₙSiX₄₋ₙ (II)
in der R'' Alkyl, Alkenyl, Aryl, Arylalkyl, Alkylaryl, Arylalkenyl oder Alkenylaryl bedeutet, wobei diese Reste durch Sauerstoff- oder Schwefelatome oder -NH-Gruppen unterbrochen sein können, X Wasserstoff, Halogen, Hydroxy, Alkoxy, Acyloxy oder die Gruppe -NR₂' (R' = Wasserstoff und/oder Alkyl) darstellt und n den Wert 1, 2 oder 3 hat,
und/oder
(c) ein organofunktionelles Silan der Formel (III)
Rₘ''(R'''Y)ₙSiX₍₄₋ₘ₋ₙ₎ (III),
in der R'', X und n die oben gegebene Bedeutung haben, R''' Alkylen, Phenylen, Alkylenphenylen oder Alkenylen bedeutet, wobei diese Reste durch Sauerstoff- oder Schwefelatome oder -NH-Gruppen unterbrochen sein können, Y Halogenatome, Hydroxy-, Mercapto-, Polyol-, z.B. Glykyl- oder Glyceryl-, gegebenenfalls substituierte Amino-, quarternäre Ammonium-, Amid-, Polyamid-, Aldehyd-, Keto-, Carboxy-, Carbonsäurealkylester-, Sulfonsäure-, Phosphorsäure-, Epoxy-, Acryloxy- oder Methacryloxygruppen darstellen und m den Wert 0, 1 oder 2 hat.

12. Zusammensetzung nach Anspruch 11, dadurch gekennzeichnet, daß sie 1 bis 99, vorzugsweise 20 bis 90, insbesondere 40 bis 80 Mol-% an Komponente (a) und entsprechend 1 bis 99, vorzugsweise 10 bis 80 und insbesondere 20 bis 60 Mol-% an Komponente (b) oder
1 bis 99, vorzugsweise 20 bis 90, insbesondere 40 bis 80 Mol-% an Komponente (a) und entsprechend 1 bis 99, vorzugsweise 10 bis 80 und insbesondere 20 bis 60 Mol-% an Komponente (c)
oder
1 bis 98, vorzugsweise 20 bis 80, insbesondere 40 bis 60 Mol-% an Komponente (a), 1 bis 98, vorzugsweise 10 bis 75 und insbesondere 15 bis 50 Mol-% an Komponente (b) und 1 bis 40, vorzugsweise 5 bis 30 und insbesondere 10 bis 25 Mol-% an Komponente (c) umfaßt.

13. Zusammensetzung nach den Ansprüchen 11 bis 12, dadurch gekennzeichnet, daß sie zusätzlich
(d) ein im Reaktionsmedium lösliches, schwer-flüchtiges Oxid eines Elements der Hauptgruppen Ia bis Va oder der Nebengruppen IVb oder Vb des Periodensystems, mit Ausnahme von Titan, Zirkon, Silicium, Germanium, Zinn und Aluminium, oder eine im Reaktionsmedium lösliche, unter den Reaktionsbedingungen ein schwerflüchtiges Oxid bildende Verbindung eines dieser Elemente
umfaßt.

14. Zusammensetzung nach Anspruch 13, dadurch gekennzeichnet, daß sie 0,1 bis 50, vorzugsweise 0,5 bis 30 und insbesondere 2 bis 20 Gew.-% an Komponente (d), bezogen auf das Gesamtgewicht der Ausgangskomponenten (a) bis (d), umfaßt.

15. Zusammensetzung nach den Ansprüchen 11 bis 14, dadurch erhältlich, daß man die Ausgangskomponenten (a), (b) und/oder (c) und gegebenenfalls (d) in den gewünschten Mengenverhältnissen, gegebenenfalls unter Einsatz von geeigneten Katalysatoren vorkondensiert, das Vorkondensat isoliert und zur Herstellung von Beschichtungslösungen einer Viskosität von etwa 5 bis 80 MPa·s in einem organischen Lösungsmittel löst.

16. Zusammensetzung nach Anspruch 15, dadurch gekennzeichnet, daß man sie mit einem ethylenisch ungesättigten copolymerisierbaren Monomeren, das mit der Vorkondensatlösung homogen mischbar oder darin löslich ist, und einem geeigneten Initiator abmischt.

17. Zusammensetzung nach Anspruch 16, dadurch gekennzeichnet, daß die Menge an ethylenisch ungesättigten Monomeren/Vernetzer 1 bis 50, vorzugsweise 10 bis 30 Gew.-%, bezogen auf das Gesamtgewicht aus Vorkondensat und ethylenisch ungesättigten Monomeren/Vernetzer, und die Menge an Initiator 0,05 bis 3 Gew.-%, bezogen auf das Gewicht der ethylenisch ungesättigten Monomeren/Vernetzer, beträgt.

## Claims

1. Use of compositions comprising
(a) at least one compound that is soluble in the reaction medium having the formula (I)
MRₓ (I),
in which M means Ti, Zr, Si, Ge, Sn or Al, R means halogen, hydroxy, alkoxy, acyloxy or a chelate ligand and x means the valency of M, and
(b) an organic silane having the formula (II)
R''ₙSiX₄₋ₙ (II)
in which R'' means alkyl, alkenyl, aryl, arylalkyl, alkylaryl, arylalkenyl or alkenylaryl, where said radicals may be interrupted by oxygen or sulphur atoms or -NH- groups, X means hydrogen, halogen, hydroxy, alkoxy, acyloxy or the group -NR₂' (R' = hydrogen and/or alkyl) and n has the value 1, 2 or 3, and/or
(c) an organofunctional silane having the formula (III)
Rₘ''(R'''Y)ₙSiX₍₄₋ₘ₋ₙ₎ (III),
in which R'', X and n have the meaning given above, R''' means alkylene, phenylene, alkylene phenylene or alkenylene, where said radicals may be interrupted by oxygen or sulphur atoms or -NH-groups, Y means halogen atoms, hydroxy, mercapto, polyol, e.g., glycyl or glyceryl, optionally substituted amino, quaternary ammonium, amide, polyamide, aldehyde, keto, carboxy, carboxylic acid alkyl ester, sulphonic acid, phosphoric acid, epoxy, acryloxy or methacryloxy groups and m has the value 0, 1, or 2,
for the preparation of coatings for dental prostheses or for the preparation of cosmetic coatings for teeth.

2. Use according to claim 1, characterised in that the compositions contain 1 to 99, preferably 20 to 90, particularly 40 to 60 mole % of component (a) and accordingly 1 to 99, preferably 10 to 80 and particularly 20 to 60 mole % of component (b)
or
1 to 99, preferably 20 to 90, particularly 40 to 80 mole % of component (a) and accordingly 1 to 99, preferably 10 to 80 and particularly 20 to 60 mole % of component (c)
or
1 to 98, preferably 20 to 80, particularly 40 to 60 mole % of component (a), 1 to 98, preferably 10 to 75 and particularly 15 to 50 mole % of component (b) and 1 to 40, preferably 5 to 30 and particularly 10 to 25 mole % of component (c).

3. Use according to claims 1 to 2, characterised in that the compositions additionally contain
(d) a sparingly volatile oxide, soluble in the reaction medium, of an element of the main groups Ia to Va or of the sub-groups IVb or Vb of the Periodic System with the exception of titanium, zirconium, silicon, germanium, tin and aluminium, or a compound of one of said elements that is soluble in the reaction medium and forms a sparingly volatile oxide under the reaction conditions.

4. Use according to claim 3, characterised in that the compositions contain 0.1 to 50, preferably 0.5 to 30 and particularly 2 to 20 % by wt. of component (d) based on the total weight of the starting components (a) to (d).

5. Use according to claims 1 to 4, characterised in that the starting components (a), (b) and/or (c) and optionally (d) are precondensed in the desired quantity ratios, optionally with the use of suitable catalysts, the precondensate is isolated and dissolved in an organic solvent in order to prepare coating solutions with a viscosity of about 5 to 80 Mpa.s.

6. Use according to claim 5, characterised in that the coatings are mixed with an ethylenically unsaturated copolymerisable monomer which is homogeneously miscible with the precondensate solution or soluble therein, and a suitable initiator.

7. Use according to claim 6, characterised in that the quantity of ethylenically unsaturated monomer/cross-linking agent is 1 to 50, preferably 10 to 30 % by wt., based on the total weight of precondensate and ethylenically unsaturated monomer/cross-linking agent, and the quantity of initiator is 0.05 to 3% by wt., based on the weight of ethylenically unsaturated monomer/cross-linking agent.

8. Use of the coating solutions obtained according to claims 5 to 7 for the preparation of coatings on dental prostheses, characterised in that the coating solutions are deposited on the dental prostheses and then cured at temperatures from room temperature to 120 °C, optionally with irradiation with visible light or UV light.

9. Use according to claim 8, characterised in that the coating solutions are deposited on dental prostheses made of plastics.

10. Use according to claim 9, characterised in that the coating solutions are deposited on plastics veneers.

11. A composition for the therapeutic coating of teeth, comprising
(a) at least one compound that is soluble in the reaction medium having the formula (I)
MRₓ (I)
in which M means Ti, Zr, Si, Ge, Sn or Al,
R means halogen, hydroxy, alkoxy, acyloxy or a chelate ligand and x means the valency of M,
and
(b) an organic silane having the formula (II)
R''ₙSiX₄₋ₙ (II)
in which R'' means alkyl, alkenyl, aryl, arylalkyl, alkylaryl, arylalkenyl or alkenylaryl, where said radicals may be interrupted by oxygen or sulphur atoms or -NH- groups, X means hydrogen, halogen, hydroxy, alkoxy, acyloxy or the group -NR₂' (R' = hydrogen and/or alkyl) and n has the value 1, 2 or 3,
and/or
(c) an organofunctional silane having the formula (III)
Rₘ''(R'''Y)ₙSiX₍₄₋ₘ₋ₙ₎ (III)
in which R'', X and n have the meaning given above, R''' means alkylene, phenylene, alkylene phenylene or alkenylene, where said radicals may be interrupted by oxygen or sulphur atoms or -NH- groups, Y means halogen atoms, hydroxy, mercapto, polyol, e.g., glycyl or glyceryl, optionally substituted amino, quaternary ammonium, amide, polyamide, aldehyde, keto, carboxy, carboxylic acid alkylester, sulphonic acid, phosphoric acid, epoxy, acryloxy or methacryloxy groups and m has the value 0, 1 or 2.

12. A composition according to claim 11, characterised in that it contains 1 to 99, preferably 20 to 90, particularly 40 to 80 mole % of component (a) and accordingly 1 to 99, preferably 10 to 80 and particularly 20 to 60 mole % of component (b), or
1 to 99, preferably 20 to 90, particularly 40 to 80 mole % of component (a) and accordingly 1 to 99, preferably 10 to 80 and particularly 20 to 60 mole % of component (c),
or
1 to 98, preferably 20 to 80, particularly 40 to 60 mole % of component (a), 1 to 98, preferably 10 to 75 and particularly 15 to 50 mole % of component (b) and 1 to 40, preferably 5 to 30 and particularly 10 to 25 mole % of component (c).

13. A composition according to claims 11 to 12, characterised in that it additionally contains
(d) a sparingly volatile oxide, soluble in the reaction medium, of an element of the main groups Ia to Va or of the sub-groups IVb or Vb of the Periodic System, with the exception of titanium, zirconium, silicon, germanium, tin and aluminium, or a compound of one of said elements that is soluble in the reaction medium and forms a sparingly volatile oxide under the reaction conditions.

14. A composition according to claim 13, characterised in that it contains 0.1 to 50, preferably 0.5 to 30 and particularly 2 to 20% by wt. of component (d), based on the total weight of the starting components (a) to (d).

15. A composition according to claims 11 to 14, obtainable in that the starting components (a), (b) and/or (c) and optionally (d) are precondensed in the desired quantity ratios, optionally with the use of suitable catalysts, the precondensate is isolated and dissolved in an organic solvent in order to prepare coating solutions with a viscosity of about 5 to 80 MPa.s.

16. A composition according to claim 15, characterised in that it is mixed with an ethylenically unsaturated copolymerisable monomer which is homogeneously miscible with the precondensate solution or soluble therein, and a suitable initiatior.

17. A composition according to claim 16, characterised in that the quantity of ethylenically unsaturated monomer/cross-linking agent is 1 to 50, preferably 10 to 30% by wt., based on the total weight of precondensate and ethylenically unsaturated monomer/cross-linking agent, and the quantity of initiator is 0.05 to 3% by wt., based on the weight of ethylenically unsaturated monomer/cross-linking agent.

## Revendications

1. Utilisation de compositions comprenant
(a) au moins un composé soluble dans le milieu réactionnel et de formule (I)
MRₓ (I)
dans laquelle M représente Ti, Zr, Si, Ge, Sn ou Al, R représente un atome d'halogène, un groupe hydroxy, alcoxy, acyloxy ou un ligand de chélation et
X représente la valence de M,
ainsi que
(b) un silane organique de formule (II)
R''ₙSiX₄₋ₙ (II)
dans laquelle R'' représente un groupe alkyle, alcényle, aryle, arylalkyle, alkylaryle, arylalcényle ou alcénylaryle, où ces radicaux peuvent être interrompus par des atomes d'oxygène ou de soufre ou encore par des groupes -NH-,
X représente un atome d'hydrogène ou d'halogène ou un groupe hydroxy, alcoxy, acyloxy ou le groupe -NR₂' (R' = hydrogène et/ou alkyle) et n a pour valeur 1, 2 ou 3,
et/ou
c) un silane organofonctionnel de formule (III)
Rₘ''(R'''Y)ₙSiX₍₄₋ₘ₋ₙ₎ (III)
dans laquelle R'', X et n ont les significations précitées, R''' représente un groupe alcylène, phénylène, alcylènephénylène ou alcényle, où ces radicaux peuvent être interrompus par des atomes d'oxygène ou de soufre ou encore par des groupes -NH-, Y représente des atomes d'halogène ou des groupes hydroxy, mercapto, polyol, (par exemple, glycyle ou glycéryle), amino éventuellement substitué, ammonium quaternaire, amide, polyamide, aldéhyde, céto, carboxy, ester alkylique d'acide carboxylique, acide sulfonique, acide phosphorique, époxy, acryloxy, ou méthacryloxy; et m a pour valeur 0, 1 ou 2
pour préparer des revêtements de prothèses dentaires ou pour préparer des revêtements cosmétiques pour dents.

2. Utilisation selon la revendication 1, caractérisée en ce que les compositions comprennent de 1 à 99 et, de préférence, de 20 à 90 et, plus particulièrement, de 40 à 80 % en moles de constituant (a) et, de manière correspondante, de 1 à 99 et, de préférence, de 10 à 80 et, plus particulièrement, de 20 à 60 % en moles de constituant (b), ou
de 1 à 99 et, de préférence de 20 à 90 et, en particulier, de 40 à 80% en moles de constituant (a) et, de manière correspondante, de 1 à 99 et, de préférence, de 10 à 80 et, plus particulièrement, de 20 à 60% en moles de constituant (c), ou
de 1 à 98 et, de préférence de 20 à 80 et, en particulier, de 40 à 60% en moles de constituant (a), de 1 à 98 et, de préférence, de 10 à 75 et, plus particulièrement de 15 à 50% en moles de constituant (b), et de 1 à 40 et, de préférence, de 5 à 30 et, plus particulièrement, de 10 à 25% en moles de constituant (c).

3. Utilisation selon les revendications 1 à 2, caractérisée en ce que les compositions comprennent en outre,
(d) un oxyde difficilement volatil et soluble dans le milieu réactionnel d'un élément des groupes principaux Ia à Va ou des groupes secondaires IVb ou Vb du système périodique, à l'exception du titane, du zirconium, du silicium, du germanium, de l'étain et de l'aluminium ou un composé de l'un de ces éléments formant un oxyde difficilement volatil dans les conditions de la réaction et soluble dans le milieu réactionnel.

4. Utilisation selon la revendication 3, caractérisée en ce que les compositions comprennent de 0,1 à 50 et,de préférence,de 0,5 à 30 et, en particulier, de 2 à 20% en poids de constituant (d), rapportés au poids total des matières premières (a) à (b).

5. Utilisation selon les revendications 1 à 4, caractérisée en ce que l'on effectue une précondensation des constituants de départ (a), (b) et/ou (c) et, éventuellement (d) dans les proportions souhaitées, éventuellement avec utilisation de catalyseurs appropriés, que l'on sépare le précondensat et que l'on dissout celui-ci dans un solvant organique pour obtenir des solutions de revêtement d'une viscosité comprise entre environ 5 et 80 MPa·s.

6. Utilisation selon la revendication 5, caractérisée en ce que l'on mélange les revêtements avec un monomère copolymérisable éthyléniquement insaturé qui est miscible de manière homogène avec la solution de précondensat ou qui y est soluble, et avec un initiateur approprié.

7. Utilisation selon la revendication 6, caractérisée en ce que la quantitié de monomères/agent réticulant éthyléniquement insaturés est comprise entre 1 et 50 et, de préférence, entre 10 et 30% en poids, rapportés au poids total de précondensat et de monomères/réticulant éthyléniquement insaturés, et que la quantité d'initiateur est comprise entre 0,05 et 3% en poids rapportés au poids de monomères/réticulant éthyléniquement insaturés.

8. Utilisation de solutions de revêtement obtenues selon les revendications 5 à 7, pour préparer des revêtements pour prothèses dentaires, caractérisée en ce que l'on applique les solutions de revêtement sur les prothèses dentaires et qu'on les durcit ensuite à des températures comprises entre la température ambiante et 120°C, éventuellement avec irradiation par la lumière visible ou la lumière ultraviolette.

9. Utilisation selon la revendication 8, caractérisée en ce que l'on applique les solutions de revêtement sur des prothèses dentaires en matière plastique.

10. Utilisation selon la revendication 9 , caractérisée en ce que l'on applique les solutions de revêtement sur des obturations en matière plastique.

11. Composition pour le revêtement thérapeutique de dents, comprenant :
(a) au moins un composé soluble dans le milieu réactionnel et de formule (I)
MRₓ (I)
dans laquelle M représente Ti, Zr, Si, Ge, Sn ou Al, R représente un atome d'halogène, un groupe hydroxy, alcoxy, acyloxy ou un ligand de chélation et
X représente la valence de M,
ainsi que
(b) un silane organique de formule (II)
R''ₙSiX₄₋ₙ (II)
dans laquelle R'' représente un groupe alkyle, alcényle, aryle, arylalkyle, alkylaryle, arylalcényle ou alcénylaryle, où ces radicaux peuvent être interrompus par des atomes d'oxygène ou de soufre ou encore par des groupes -NH-,
X représente un atome d'hydrogène ou d'halogène ou un groupe hydroxy, alcoxy, acyloxy ou le groupe -NR₂. (R' = hydrogène et/ou alkyle) et n a pour valeur 1, 2 ou 3,
et/ou
(c) un silane organofonctionnel de formule (III)
Rₘ''(R'''Y)ₙSiX₍₄₋ₘ₋ₙ₎ (III)
dans laquelle R'', X et n ont les significations précitées, R''' représente un groupe alcylène, phénylène, alcylènephénylène ou alcényle, où ces radicaux peuvent être interrompus par des atomes d'oxygène ou de soufre ou encore par des groupes -NH-, Y représente des atomes d'halogène ou des groupes hydroxy, mercapto, polyol, (par exemple, glycyle ou glycéryle), amino éventuellement substitué, ammonium quaternaire, amide, polyamide, aldéhyde, céto, carboxy, ester alkylique d'acide carboxylique, acide sulfonique, acide phosphorique, époxy, acryloxy, ou méthacryloxy; et m a pour valeur 0, 1 ou 2.

12. Composition selon la revendication 11, caractérisée en ce que les compositions comprennent de 1 à 99 et, de préférence, de 20 à 90 et, plus particulièrement, de 40 à 80 % en moles de constituant (a) et, de manière correspondante, de 1 à 99 et, de préférence, de 10 à 80 et, plus particulièrement, de 20 à 60 % en moles de constituant (b), ou
les compositions comprennent de 1 à 99 et, de préférence de 20 à 90 et, plus particulièrement, de 40 à 80% en moles de constituant (a) et, de manière correspondante, de 1 à 99 et, de préférence, de 10 à 80 et, plus particulièrement, de 20 à 60% en moles de constituant (c), ou les compositions comprennent de 1 à 98 et, de préférence de 20 à 80 et, en particulier, de 40 à 60% en moles de constituant (a), de 1 à 98 et, de préférence, de 10 à 75 et, plus particulièrement de 15 à 50% en moles de constituant (b), et de 1 à 40 et, de préférence, de 5 à 30 et, plus particulièrement, de 10 à 25% en moles de constituant (c).

13. Composition selon les revendications 11 et 12, caractérisées en ce qu'elle comprend en outre
(d) un oxyde difficilement volatil et soluble dans le milieu réactionnel d'un élément des groupes principaux Ia à Va ou des groupes secondaires IVb ou Vb du système périodique, à l'exception du titane, du zirconium, du silicium, du germanium, de l'étain, de l'aluminium ou des composés de l'un de ces éléments formant un oxyde difficilement volatil dans les conditions de la réaction et soluble dans le milieu réactionnel.

14. Composition selon la revendication 13, caractérisée en ce qu'elle comprend de 0,1 à 50 et, de préférence, de 0,5 à 30 et, en particulier, de 2 à 20% en poids de constituant (d) rapportés au poids total des matières premières (a) à (d).

15. Composition selon les revendications 11 à 14, pouvant être obtenue en précondensant les constituants de départ (a), (b) et/ou (c), éventuellement (d) dans la proportion souhaitée, éventuellement avec utilisation de catalyseurs appropriés, en séparant le précondensat et en le dissolvant dans un solvant organique pour obtenir des solutions de revêtement dont la viscosité est comprise entre environ 5 et 80 MPa·s.

16. Composition selon la revendication 15, caractérisée en ce qu'on la mélange avec un monomère polymérisable éthyléniquement insaturé qui est miscible de manière homogène avec la solution de précondensat qui y est soluble et avec un initiateur approprié.

17. Composition selon la revendication 16, caractérisée en ce que la quantité de monomères/agent réticulant éthyléniquement insaturés est comprise entre 1 et 50 et, de préférence, entre 10 et 30% en poids rapportés au poids total de précondensat et de monomères/agent réticulant éthyléniquement insaturés, et que la quantité d'initiateur est comprise entre 0,05 et 3% en poids rapportée au poids de monomères/réticulant éthyléniquement insaturés.
